# EUROPEAN PATENT APPLICATION

(11) **EP 3 296 842 A1**
(43) Date of publication of application: **21.03.2018**
(21) Application number: 16197542.0
(22) Date of filing: 07.11.2016
(51) Int. Cl.: G06F 3/01, G06F 3/0484

(54) **SYSTEM AND METHOD FOR ADAPTING A DISPLAY ON AN ELECTRONIC DEVICE**

(30) Priority: 20.09.2016 IN 201641032077
(71) Applicant: Wipro Limited, 560 035 Karnataka (IN)
(72) Inventor: NANDARAGI, Prasanna, 560078 Bangalore (IN)
(74) Representative: Finnegan Europe LLP

(57) **Abstract**

A technique is provided for adapting a display on an electronic device. The technique includes displaying a first user interface comprising a first plurality of graphical prompts to a user, wherein the first plurality of graphical prompts are highlighted sequentially, detecting one or more pre-defined eye-blinking gestures of a user to select a highlighted graphical prompt of the first user interface, and adapting the first user interface, to display a second user interface in response to detecting the one or more pre-defined eye-blinking gestures of the user.

## Description

### Technical Field

This disclosure relates generally to display devices, and more particularly to system and method for adapting a display on an electronic device.

### Background

Advancements in the field of display devices have led to development of various displays that can be configured for communicating with patients rendered incapacitated due to critical illness or disability caused by accidents. Because such incapacitated patients have a limited scope for interacting with the people such as doctors, nurses, family members, the aforementioned displays are aimed at assisting the patients in communicating their requirements. Such an assistance is based on tracking movements associated with the eyes of the patient, such as eye ball movement, gaze detection, and the like.

In certain scenarios, an intermediary in the form of a nurse, doctor, or a family member may have to be present in order to elicit the requirements from the patient. Such requirements may be elicited with the help of menu displayed on a display. In certain other scenarios, the patient may directly communicate the requirements based on gaze based navigation gestures. However, the aforementioned methods either require the presence of the third person in the form of the intermediary or such methods are based on gaze of the patient. Clearly such methods are resource intensive or are prone to errors due to incorrect gaze tracking. Furthermore, such methods cause inconvenience to the patient as it is difficult for an incapacitated patient to navigate the displayed menu based on gaze based gestures.

It is therefore desirable to provide a stress-free and a convenient mechanism to the patient for communicating the requirements to the caretakers. It is also desirable that such a mechanism includes enhanced usability aspects that enable the patient to conveniently navigate the entire menu based on a limited set of gestures.

### SUMMARY

In one embodiment, a method of adapting a display on an electronic device is disclosed. In one example, the method comprises displaying a first user interface comprising a first plurality of graphical prompts to a user. The first plurality of graphical prompts may be highlighted sequentially. The method further comprises detecting one or more pre-defined eye-blinking gestures of the user for selection of a highlighted graphical prompt of the first user interface. The method further comprises adapting the first user interface for displaying a second user interface, in response to the detection of the one or more pre-defined eye-blinking gestures of the user.

In another embodiment, a system is disclosed for adapting a display on an electronic device is disclosed. The system comprises a processor and a memory communicatively coupled to the processor. The memory stores processor-executable instructions, which, on execution, cause the processor to display a first user interface comprising a first plurality of graphical prompts to a user. The first plurality of graphical prompts may be highlighted sequentially. The processor-executable instructions, on execution, further cause the processor to detect one or more pre-defined eye-blinking gestures to a user for selection of a highlighted graphical prompt of the first user interface. The processor-executable instructions, on execution, further cause the processor to adapt the first user interface for displaying a second user interface, in response to the detection of the one or more pre-defined eye-blinking gestures of the user.

In yet another embodiment, a non-transitory computer-readable medium storing computer-executable instructions for adapting a display on an electronic device is disclosed. In one example, the stored instructions, when executed by a processor, cause the processor to perform operations comprising displaying a first user interface comprising a first plurality of graphical prompts to a user. The first plurality of graphical prompts may be highlighted sequentially. The operations further comprise detecting one or more pre-defined eye-blinking gestures to the user for selection of a highlighted graphical prompt of the first user interface. The operations further comprise adapting the first user interface for displaying a second user interface, in response to the detection of the one or more pre-defined eye-blinking gestures of the user.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles.
**FIG. 1** is a block diagram of an exemplary network environment for adapting a display on an electronic device over a communication network, in accordance with some embodiments of the present disclosure.
**FIG. 2** is a block diagram of exemplary system for adapting a display on an electronic device, in accordance with some embodiments of the present disclosure.
**FIG. 3A** illustrates a first exemplary scenario of adapting a display on an electronic device, in accordance with some embodiments of the present disclosure.
**FIG. 3B** illustrates a second exemplary scenario of adapting a display on an electronic device, in accordance with some embodiments of the present disclosure.
**FIG. 4** is a flow diagram of a detailed exemplary process for adapting a display on an electronic device, in accordance with some embodiments of the present disclosure.
**FIG. 5** is a block diagram of an exemplary computer system for implementing embodiments consistent with the present disclosure.

### DETAILED DESCRIPTION

Exemplary embodiments are described with reference to the accompanying drawings. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

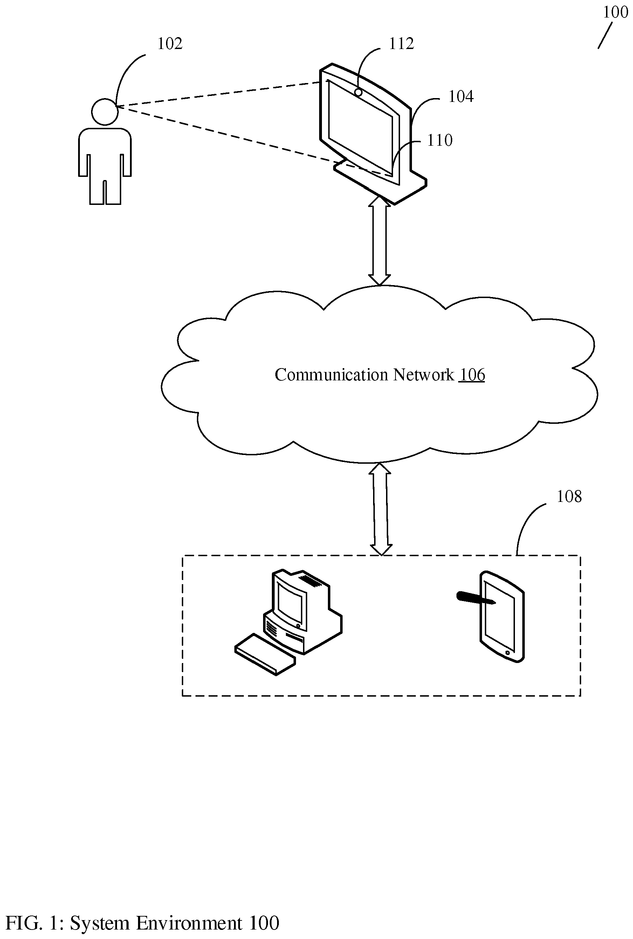

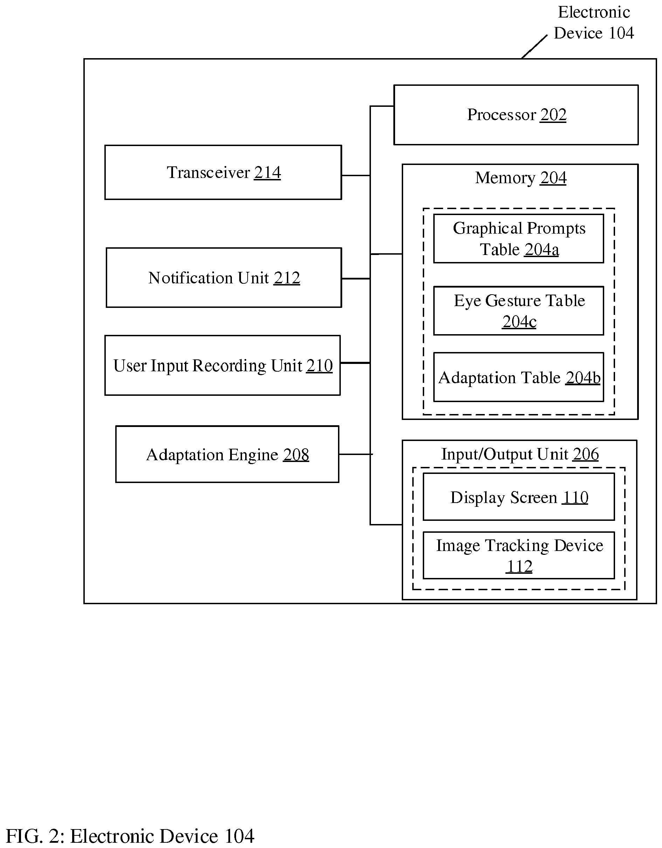

Referring now to FIG. 1, an exemplary network environment 100 for adapting a display on an electronic device is illustrated in accordance with some embodiments of the present disclosure. As will be described in greater detail in conjunction with FIG. 2, the network environment 100 displays a first user interface comprising a first plurality of graphical prompts to a user, such as the user 102. The first plurality of graphical prompts may be highlighted sequentially. The system may further detect one or more pre-defined eye-blinking gestures of the user 102 for selection of a highlighted graphical prompt of the first user interface. The system may further adapt the first user interface for displaying a second user interface, in response to the detection of the one or more pre-defined eye-blinking gestures of the user 102.

The network environment 100 includes a network of computing devices (e.g., a computer, a server, a digital device, a router, a modem, a bridge, a switch, etc.) for sending or receiving various data. In one embodiment, the network environment 100 includes a user 102 having an associated electronic device 104, and a one or more remote devices, such as the remote device 108. The electronic device 104 and the remote device 108 may be in communication with each other over a wired or wireless communication network 106. Each of the computing devices further includes one or more processors and a computer-readable medium (e.g., a memory). The computer-readable storage medium stores instructions that, when executed by the one or more processors, cause the one or more processors to perform data reception and transmission in accordance with aspects of the present disclosure. The computer-readable storage medium may also store various data (e.g., optimal network path, optimal packet size, data packets, current packet size of a node, etc.) that may be captured, processed, and/or required by the network environment 100.

The electronic device 104 may include suitable logic, circuitry, interfaces, and/or code for displaying one or more user interfaces to the user 102 on a display screen 110. The one or more user interfaces may include the first user interface and the second user interface. Examples of implementation of the display screen 110 of the electronic device 104 may include, but are not limited to, a Liquid Crystal Display (LCD) display, a Light Emitting Diode (LED) display, an Organic LED (OLED) display technology. The electronic device 104 may further include an image tracking device 112 in the form of a camera for tracking the eye-movements of the user 102. In an implementation, the image tracking device 112 may be integrated internally into the electronic device 104. In another implementation, the image tracking device 112 may be external to the electronic device 104 and may be communicatively coupled via the communication network 106.

The communication network 106 may include a medium through which the electronic device 104 and the remote device 108 present in the network environment 100 may communicate with each other. Examples of the communication network 106 may include, but are not limited to, the Internet, a cloud network, a Wireless Fidelity (Wi-Fi) network, a Wireless Local Area Network (WLAN), a Local Area Network (LAN), a telephone line (POTS), Long Term Evolution (LTE), and/or a Metropolitan Area Network (MAN). Various devices in the exemplary network environment 100 may be configured to connect to the communication network 106, in accordance with various wired and wireless communication protocols. Examples of such wired and wireless communication protocols may include, but are not limited to, Transmission Control Protocol and Internet Protocol (TCP/IP), User Datagram Protocol (UDP), Hypertext Transfer Protocol (HTTP), File Transfer Protocol (FTP), Zigbee, EDGE, infrared (IR), IEEE 802.11, 802.16, cellular communication protocols, and/or Bluetooth (BT) communication protocols.

The remote device 108 may include suitable logic, circuitry, interfaces, and/or code for displaying one or more notifications generated at the electronic device 104 based on the one or more user inputs provided by the user 102. Examples of the implementation of the remote device 108 include, but are not limited to, a network of personal computers, a Personal Digital Assistant (PDA) devices, a laptop, or a tablet.

In operation, the electronic device 104 may display a first user interface on the display screen 110. The displayed first user interface may include a first plurality of graphical prompts. In an embodiment, the displayed first plurality of graphical prompts may correspond to menu items pre-configured into the system. The menu items may include, but are not limited to, a request to contact a person, a request to order one or more consumables, a request to express physical or a mental state, etc. In an embodiment, the menu items may be configured into the system by a system administrator. In another embodiment, the menu items may be dynamically determined from the user based on at least the displayed first plurality of graphical prompts. The electronic device 104 may further highlight each of the displayed first plurality of graphical prompts sequentially for a first pre-defined time interval. In an embodiment, the highlighting of the first plurality of graphical prompts may be based on visual highlighting of the graphical prompts on the display screen 110. In another embodiment, the highlighting of the first plurality of graphical prompts may be based on audio output produced by the electronic device 104, for each of the displayed graphical prompt.

The electronic device 104 may further perform automatic transitions from the first user interface to a second user interface. Such automatic transitions may be performed based on a second pre-defined time interval. In an embodiment, the automatic transitions may include, but are not limited to, a vertical scroll from the first user interface to the second user interface, a horizontal scroll from the first user interface to the second user interface, a fade-in fade-out transition, a flash transition, etc. Further, the second user interface may include a second plurality of graphical prompts, each of which may be highlighted sequentially for the first predefined time interval. In an embodiment, the second plurality of graphical prompts may correspond to a selected graphical prompt from the first plurality of graphical prompts. In an instance, based on the displayed second plurality of graphical prompts, the user 102 may provide another input for selection of a graphical prompt from the second plurality of graphical prompt. In another instance, the second plurality of graphical prompts may correspond to a confirmation selection made by the user 102. In such an instance, the highlighting of the second plurality of the graphical prompts may not be required to be performed by the electronic device 104.

The electronic device 104, via the image tracking device 112 may be configured to track the eye-movements of the user 102. In an embodiment, the image tracking device 112 may detect one or more pre-defined eye blinking gestures of the user 102 to select a highlighted graphical prompt of the displayed first user interface. In an embodiment, the one or more pre-defined eye-blinking gesture may include, but are not limited to, a blink of one or both eyes of the user 102 for a pre-defined count, closing one or both eyes of the user 102 for a predefined duration, a combination of blink of the eyes of the user 102. Furthermore, in an embodiment, the aforementioned gestures may include a first pre-defined eye-blinking gesture to stop the highlighting of the graphical prompts followed by a second pre-defined eye-blinking gesture to select the highlighted graphical prompt at which the highlighting has been stopped.

The electronic device 104 may further adapt the first user interface to display the second user interface as a response to the detected one or more predefined eye blinking gestures. In an embodiment, such an adaptation includes controlling display properties of the first plurality of the graphical prompts and the second plurality of the graphical prompts. The examples of the display properties may include, but are not limited to, a time interval of the sequential highlighting of the first plurality of the graphical prompts, a time interval of a sequential highlighting of the second plurality of the graphical prompts, a size of the first plurality of the graphical prompts or the second plurality of the graphical prompts, a display duration of the first plurality of the graphical prompts or the second plurality of the graphical prompts, and a level of brightness of the display unit.

In an embodiment, the electronic device 104 may further store the detected one or more one or more pre-defined eye-blinking gestures of the user 102. Such a storage operation may be performed in a memory associated with the electronic device 104. In an embodiment, along with the detected pre-defined eye-blinking gestures of a user 102 at a point of time, the adaptation of the first user interface to the second user interface may be further based on a history of the detected predefined eye-blinking gestures of the user 102. A person of ordinary skill in the art will appreciate that the aforementioned system may comprise a plurality of other interfaces, such as a third user interface, a fourth interface, and the like. In an embodiment, the transition from the first user interface to one of the plurality of user interfaces may be based on adaptation performed by the electronic device 104. Further, such an adaptation may be based on the detected one or more predefined eye-blinking gestures of the user 102.

In an embodiment, the electronic device 104 may generate one or more notifications based on the detected one or more pre-defined eye-blinking gestures of the user. The notifications may be transmitted to the remote device 108. In some embodiments, the notifications may be generated as soon as the user commences selection of the displayed first plurality of graphical prompts. In some other embodiments, the notifications may be generated based on various medical events (such as a medical emergency) associated with the user. The notifications may also be generated based on a physical or a mental state of the user, a status of request placed by the user, and a selection of the one or more graphical prompts from the first plurality of graphical prompts. In an embodiment, the electronic device 104 may determine the medical emergency associated with the user 102 based on at least a comparison of average time taken by the user to select a highlighted graphical prompt, with a pre-defined time threshold. In instances, when the user inputs corresponding to the selection of one or more displayed graphical prompt are not provided within the pre-defined time threshold, the electronic device 104 may infer that a medical emergency associated with the user has occurred and may generate one or more notifications that may be transmitted to the remote device 108, via the communication network 106.

It should be noted that the various modules described above may be implemented in programmable hardware devices such as programmable gate arrays, programmable array logic, programmable logic devices, and so forth. Alternatively, the various modules described above may be implemented in software for execution by various types of processors. An identified module of executable code may, for instance, include one or more physical or logical blocks of computer instructions which may, for instance, be organized as an object, procedure, function, engine, or other construct. Nevertheless, the executables of an identified module need not be physically located together, but may include disparate instructions stored in different locations which, when joined logically together, include the module and achieve the stated purpose of the module. Indeed, a module of executable code could be a single instruction, or many instructions, and may even be distributed over several different code segments, among different applications, and across several memory devices.

As will be appreciated by one skilled in the art, a variety of processes may be employed for transmitting data over a communication network. For example, the exemplary network environment 100 may transmit data over a communication network by the processes discussed herein. In particular, as will be appreciated by those of ordinary skill in the art, control logic and/or automated routines for performing the techniques and steps described herein may be implemented by the network environment 100, either by hardware, software, or combinations of hardware and software. For example, suitable code may be accessed and executed by the one or more processors on the network environment 100 to perform some or all of the techniques described herein. Similarly, application specific integrated circuits (ASICs) configured to perform some or all of the processes described herein may be included in the one or more processors on the network environment 100.

FIG. 2 is a block diagram of exemplary system for adapting a display on an electronic device, in accordance with some embodiments of the present disclosure. FIG. 2 is explained in conjunction with elements from FIG. 1. With reference to FIG. 2, there is shown the electronic device 104. The electronic device 104 may include one or more processors, such as a processor 202, a memory 204, an input/output (I/O) unit 206, an adaptation engine 208, a user input recording unit 210, a notification unit 212, and a transceiver 214. The processor 202 may be communicatively coupled to the memory 204, the I/O unit 206, the adaptation engine 208, the user input recording unit 210, the notification unit 212, and the transceiver 214. In an embodiment, the electronic device 104 may be communicatively coupled to the one or more remote devices, such as the remote device 108 through the communication network 106, via the transceiver 214.

The processor 202 may include suitable logic, circuitry, interfaces, and/or code that may be configured to execute a set of instructions stored in the memory 204. The processor 202 may be configured to track the eye-movements of the user 102 and adapt the display screen 110 of the electronic device 104 by execution of one or more tracker algorithms, stored in the memory 204 of the electronic device. Examples of the processor 202 may be an X86-based processor, a Reduced Instruction Set Computing (RISC) processor, an Application-Specific Integrated Circuit (ASIC) processor, a Complex Instruction Set Computing (CISC) processor, and/or other processors.

The memory 204 may include suitable logic, circuitry, and/or interfaces that may be configured to store a machine code and/or a computer program with at least one code section executable by the processor 202. In an embodiment, the memory 204 may be configured to store detected one or more one or more predefined eye-blinking gestures of the user 102. Further, the memory 204 may be configured to store one or more relationship tables, such as a graphical prompts table 204a, an eye gesture table 204b, and an adaptation table 204c. The memory may be further configured to store one or more configurable parameters that include, but are not limited to, a first pre-defined time interval for highlighting the graphical prompts, a second pre-defined time interval for transitioning from a first interface to a second interface, pre-defined time threshold for determining medical emergency. Examples of implementation of the memory 204 may include, but are not limited to, Random Access Memory (RAM), Read Only Memory (ROM), Hard Disk Drive (HDD), and/or a Secure Digital (SD) card.

The I/O unit 206 may include suitable logic, circuitry, interfaces, and/or code that may be configured to track eye-movements of the user 102 via the image tracking device 112, and provide an output to the user via the display screen 110. The I/O unit 206 may include various input and output devices that may be configured to communicate with the processor 202. The electronic device 104 may display one or more interfaces to the user 102 via the display screen 110. Examples of the display screen 110 have been disclosed in FIG. 1. The electronic device 104 may further track the eye-movements of the user 102 based on the image tracking device 112. The image tracking device 112 of the I/O unit 206 may be equipped with a photographic optical system, such as a photographic lens and/or a zoom lens, as well as one or more image sensors, such as a charge-coupled device (CCD) or a complementary metal-oxide-semiconductor (CMOS). Examples of implementation of the image tracking device 112 may include, but are not limited to, a digital camera, a camera embedded in a personal digital assistant (PDA), a video camera, and/or a motion camera. A person of ordinary skill in the art will appreciate that the I/O unit may further include one or more audio based output devices for enabling communication with the user 102.

The adaptation engine 208 may include suitable logic, circuitry, interfaces, and/or code that may be configured to adapt a displayed first user interface to a second user interface based on the detected one or more pre-defined eye-blinking gestures of the user 102. The adaptation may be further based on the relationship tables stored in the memory 204. The adaptation engine 208 may be implemented as one or more processors, based processor technologies known in the art.

The user input recording unit 210 may include suitable logic, circuitry, interfaces, and/or code that may be configured to store the detected one or more pre-defined eye-blinking gestures of the user 102 in the memory 204. In an embodiment, such a storing of the gestures facilitates the adaptation of the user interfaces. The user input recording unit 210 may be implemented as one or more processors, based processor technologies known in the art.

The notification unit 212 may include suitable logic, circuitry, interfaces, and/or code that may be configured to generate one or more notifications based on comparison of the average time taken by the user to select a highlighted graphical prompt, with a pre-defined time threshold stored in the memory 204. The generated one or more notifications may be transmitted to the remote device 108, via the transceiver 214. The notification unit 212 may be implemented as one or more processors, based processor technologies known in the art.

The transceiver 214 may include suitable logic, circuitry, interfaces, and/or code that may be configured to communicate with another electronic device 104 or remote device 108, via a communication network 106. The transceiver 214 may implement known technologies to support wired or wireless communication. The transceiver 214 may include, but is not limited to, an antenna, a radio frequency (RF) transceiver, one or more amplifiers, a tuner, one or more oscillators, a digital signal processor, a coder-decoder (CODEC) chipset, a subscriber identity module (SIM) card, and/or a local buffer. The transceiver 214 may communicate via wireless communication with networks, such as the Internet, an Intranet and/or a wireless network, such as a cellular telephone network, a wireless local area network (LAN) and/or a metropolitan area network (MAN). The wireless communication may use any of a plurality of communication standards, protocols and technologies, such as Global System for Mobile Communications (GSM), Enhanced Data GSM Environment (EDGE), wideband code division multiple access (W-CDMA), code division multiple access (CDMA), Long Term Evolution (LTE), time division multiple access (TDMA), Bluetooth, Wireless Fidelity (Wi-Fi) (such as IEEE 802.11a, IEEE 802.11b, IEEE 802.11g and/or IEEE 802.11n), voice over Internet Protocol (VoIP), Wi-MAX, a protocol for email, instant messaging, and/or Short Message Service (SMS).

In operation, the processor 202 in conjunction with the I/O unit 206 may be configured to display a first user interface on the display screen 110 to a user, such as the user 102. The displayed first user may include a first plurality of graphical prompts. In an embodiment, the displayed first plurality of graphical prompts may correspond to the menu items as discussed in FIG. 1. The processor 202 may be further configured to highlight each of the first plurality of graphical prompts in a pre-defined sequence that may be stored in the memory 204. In an embodiment, such a pre-defined sequence may be based on the graphical prompt table 204a stored in the memory 204. Such a table may define the relationship between a type of a graphical prompt, such as a category or a topic of a graphical prompt, with display properties of a graphical prompt. In an embodiment, the type of a graphical prompt may correspond to a question for which an answer in the form of a "yes" or "no" response is expected from the user 102. In another embodiment, the type of a graphical prompt may correspond to an icon (corresponding to a service) that can be selected by the user 102 The display properties of a graphical prompt may include, but are not limited to, a sequential order in which the first plurality of graphical prompts are to be highlighted, the first pre-defined time interval for which a graphical prompt is highlighted, a count of the number of times the sequential highlighting of a plurality of graphical prompts of a user interface is to be performed, time interval of the sequential highlighting of a graphical prompts, a color of graphical prompt, a size of a graphical prompt, a display duration of a graphical prompt, a level of brightness of the display screen 110 and/or one or more multimedia properties associated with a graphical prompt. An example of the graphical prompts table has been illustrated in Table 1.

**TABLE 1**

| **Graphical Prompt Types** | **Graphical Prompt Properties** |
|---|---|
| Question 1 | Display Duration. |
| | Font Size |
| | Type of animation |
| | Color Scheme |
| | .... |
| | .... |
| **Contact** | Sequential order |
| - Family | Color Scheme |
| - Friends | Contact Picture Location |
| .... | .... |
| | .... |
| | .... |
| **Express feeling** | .... |
| - Pain | |
| - Hungry | |

As is illustrated in Table 1, a graphical prompt corresponding to a request to place a call may be associated with one or more display properties. Such display properties may include a sequential order in which the contacts are highlighted. For example, frequently called contacts may appear higher up in the sequential order as compared to less frequently accessed contacts. Further, the one or more display properties may include pictures of the contact for identification. Similarly, the one or more display properties corresponding to a graphical prompt for an exemplary "Question 1" may include a display duration, a type of animation, and a font size, and/or a color scheme associated with the graphical prompt.

In an embodiment, the stored pre-defined sequence of highlighting the graphical prompts may be associated with a pre-stored profile of a user, such as the user 102. Therefore, in such an embodiment, the sequence of highlighting the first plurality of graphical prompts may be customized based on medical condition of the user 102. For example, when the user 102 corresponds to a critically ill patient, the graphical prompts for requesting medical services, or a request to express physical or a mental state, may be highlighted before highlighting a graphical prompt for requesting one or more consumables.

Subsequent to displaying the first user interface, the processor 202 may be configured to track the eye-movements of the user 102, based on the image tracking device 112 of the I/O unit 206. Such a tracking may be performed to detect one or more pre-defined eye-blinking gestures of the user for selection of a highlighted graphical prompt of the displayed first user interface. In an embodiment, the one or more pre-defined eye-blinking gestures may include, but are not limited to, a blink of one or both eyes of the user 102 for a pre-defined count, closing one or both eyes of the user 102 for a pre-defined duration, a combination of blink of the eyes of the user 102. In an embodiment, the processor 202 may be configured to display a list of the one or more pre-defined eye-blinking gestures to the user 102, on the display screen 110. In an embodiment, the one or more pre-defined eye-blinking gestures may correspond to a user input to halt the sequential highlighting of the graphical prompts. In another embodiment, the one or more pre-defined eye-blinking gestures may correspond to a "yes" or a "no" response for selection of a graphical prompt. A person of ordinary skill in the art will appreciate that in an embodiment, the one or more pre-defined eye-blinking gestures may be dynamically configured by a user of the electronic device 104, based on the severity of the medical condition of the user.

Once one or more of the predefined eye-blinking gestures of the user are detected, the processor 202 may refer to the eye gesture table 204b stored in the memory 204 to determine what actions to take based on the eye-blinking gesture. An eye gesture table 204b may define a relationship between categories or topics of graphical prompts (as described in detail with respect to Table 1), the one or more pre-defined eye-blinking gestures of the user 102, and a response corresponding to the one or more pre-defined eye-blinking gestures. An example of the eye gesture table has been illustrated in Table. 2.

**TABLE 2**

| **Graphical Prompt Types** | **Eye Gesture** | **User Response** |
|---|---|---|
| Question 1 | One blink or Double blink | YES or NO |
| Question x | One blink or double blink or long blink (more than 5 seconds) or three blinks | Option 1 or Option 2 or Option 3 or Option 4 |
| **Contact** | One blink or Double | YES/NO |
| - Family | Blink | YES/NO |
| - Friends | One blink or Double | YES/NO |
| | Blink | YES/NO |
| **Express feeling** | Long blink or three | YES/NO |
| - Pain | blink | YES/NO |
| - Hungry | .............. | YES/NO |
| | .......... | Response X or |
| | Eye Gesture X or Eye | Response Y |
| **Other** | Gesture Y | Response M or |
| - Topic A | Eye Gesture M or Eye | Response N |
| - Topic B | Gesture N | |
| Do you want new topic | One blink or Double link | YES/NO |
| Pain score | One blink or double blink or three blink | 1 or 2 or 3 |

As is illustrated in Table 2, for a graphical prompt corresponding to an exemplary "Question 1", acceptable eye gesture may include "single blink" or a "double blink". Further, each of the eye gestures may be mapped to a user response. For example, a "single blink" may correspond to a user response "YES", and a "double blink" may correspond to a user response "NO". Similarly, for other graphical prompts, such as the one that corresponds to "Question X", options such as "Option 1", "Option 2", "Option 3", or "Option 4" be selected based on various eye gestures, such as "single blink", "double blink", "long blink", or "three blinks". Furthermore, in an embodiment, the aforementioned eye gestures may include a first pre-defined eye-blinking gesture to stop the highlighting of the graphical prompts followed by a second pre-defined eye-blinking gesture to select the highlighted graphical prompt at which the highlighting has been stopped.

Based on the detected one or more eye-blinking gestures of the user 102, and the relationship defined in the eye gesture table 204b, the processor 202 may be configured to instruct the adaptation engine 208 to adapt the displayed first user interface to a second user interface. The second user interface may include a second plurality of graphical prompts, each of which may be highlighted sequentially for the first pre-defined time interval. In an embodiment, the second plurality of graphical prompts may correspond to a selected graphical prompt from the first plurality of graphical prompts. In an instance, based on the displayed second plurality of graphical prompts, the user 102 may provide another input for selection of a graphical prompt from the second plurality of graphical prompt. In another instance, the second plurality of graphical prompts may correspond to a confirmation selection made by the user 102. In an embodiment, the highlighting of the second plurality of the graphical prompts may not be required to be performed by the electronic device 104 when the second plurality of graphical prompts correspond to the latter instance.

In an embodiment, the adaptation may be based on the adaptation table 204c stored in the memory 204. Such an adaptation table 204c may define a relationship between a response of a user 102 or an event associated with the user 102, with the type of adaptation that is to be performed by the adaptation engine 208. In an embodiment, such adaptions may include controlling the aforementioned display properties of the first plurality of the graphical prompts and the second plurality of the graphical prompts. An example of the adaptation table 204c has been illustrated in Table. 3.

**TABLE 3**

| **Event type** | **Adaptation type** |
|---|---|
| If user response to Question 1 is yes | Modify hierarchy |
| User has not responded for "n" consecutive numbers of graphical prompts | Increase font size of Graphical prompts |
| Average time taken by user to respond to graphical display is more than threshold time | Extend display time of each graphical prompts. |
| | Example. Extend from 1 min to 3 min. |
| User has not responded to a graphical prompt | Repeat the particular prompt "n" number of times |
| User has not responded for "n" consecutive numbers of graphical prompts | Prioritized the graphical prompt and reduce scrolling path |

As is illustrated in Table 3, based on the detected eye gesture of the user 102, and event corresponding to the eye gesture, the adaptation engine 208 may refer to the aforementioned adaptation table 204c, stored in the memory 204. Each row of the adaptation table maps an event with an adaptation type that may be performed by the adaptation engine 208. For example, when the user response to "Question 1" is yes, the adaptation engine may be configured to modify the hierarchy of the subsequent user interface (the second user interface) that may be displayed. Similarly, in a scenario when a user response is not detected for a predefined number of highlighted graphical prompts, the adaptation engine 208 may be configured to increase the first pre-defined time interval for highlighting a graphical prompt. Other listed examples include changing the display properties of the graphical prompts based on the detected events.

In an embodiment, the adaptation of the first user interface to the second user interface may include modifying the hierarchy of the graphical prompts of the first plurality of graphical prompts and the second plurality of graphical prompts. Such a hierarchy may be defined in the adaptation table 204c. The modification of the hierarchy may include displaying the second plurality of graphical prompts for which a likelihood of selection by the user 102 is greater than a pre-defined likelihood. The determination of such a likelihood of selection of a graphical prompt of the second plurality of graphical prompts, may be based on one or more historical inputs associated with the user 102. Such historical inputs may be stored in the memory 204. Further, such historical inputs may correspond to previously selected graphical prompts by the user 102. In an embodiment, the aforementioned storing of each of the selected graphical prompts may be performed by the user input recording unit 210.

In an embodiment, the adaptation engine 208 may be configured to automatically transition the display screen 110 from the first user interface to the second user interface. Such transition from the first user interface to the second user interface may be performed when the one or more pre-defined eye-blinking gestures of the user 102 is not detected within a second pre-defined time interval. It may be noted that the aforementioned transition from the first user interface to the second user interface may be performed only when each of the first plurality of graphical prompts are highlighted without detection of user input within the second pre-defined time interval.

In an embodiment, the aforementioned transition from the first user interface to the second user interface may include, but is not limited to, a vertical scrolling from the first user interface to the second user interface, a horizontal scrolling from the first user interface to the second user interface, a fade-in fade-out transition from the first user interface to the second user interface, a flash transition from the first user interface to the second user interface, a dissolve transition from the first user interface to the second user interface, a wipe transition from the first user interface to the second user interface, and an iris transition from the first user interface to the second user interface.

In an embodiment, the adaptation engine 208 may be configured to modify the navigation path from the first user interface to a plurality of second user interfaces based on the relationships defined in the adaptation table 204c. For example, the first user interface may correspond to eliciting response from the user 102, corresponding to selection of high priority services. Such a priority to the services may be assigned by the processor 202 based on at least the historical user inputs of the user 102, the medical condition of the user 102, or the environment conditions (such as time of day) of the user 102. Based on the detection of one or more pre-defined eye-blinking gestures of the user 102, the adaptation engine may modify the navigation path to display a second user interface comprising services that may have the next highest priority. As an illustration, when the time of day corresponds to morning time, based on selection corresponding to ordering food item from the first user interface, the adaptation engine 208 may be configured to present the second user interface that includes the second plurality of graphical prompts corresponding to breakfast food items. Similarly, when the time of day corresponds to night time, based on selection corresponding to ordering food item from the first user interface, the adaptation engine 208 may be configured to present the second user interface that includes the second plurality of graphical prompts corresponding to dining food items. A person of ordinary skill in the art will appreciate that aforementioned adaptations are performed by the adaptation engine 208 to reduce the number of interactions required by the user 102 to request an intended service.

In an embodiment, based on the detection of one or more pre-defined eye-blinking gestures of the user 102, the adaptation engine 208 in conjunction with the I/O unit 206, may provide an option to dynamically determine an intended service. Such dynamically determined services may correspond to services that are not defined in the relationship tables stored in the memory 204. For example, based on a selection of a graphical prompt to place a call, the adaptation engine 208 may display a second plurality of graphical prompts that includes an option to dynamically determine a contact information of a contact to which the call is to be placed, from the user 102.

In an embodiment, based on the detected one or more pre-defined eye-blinking gestures of the user 102, the processor 202 may instruct the notification unit 212 to generate one or more notifications. Such one or more notifications may correspond to one or more of a medical event associated with the user, a manual or automated communication corresponding to a physical or a mental state of the user, a status of request placed by the user, and a selection of the one or more graphical prompts from the first plurality of graphical prompts. In another embodiment, the one or more notifications may be generated when a first input comprising one or more pre-defined eye-blinking gestures are detected. In an embodiment, the one or more notifications corresponding to the medical event (such as a medical emergency) may be generated based on a comparison of average time taken by the user to select a highlighted graphical prompt, with a predefined time threshold stored in the memory 204. The notification unit 212 may be configured to transmit the generated one or more notifications to the remote device 108, via the transceiver 214. Further, in another embodiment, the notification unit 212 may be configured to store the generated one or more notifications in the memory 204. In an embodiment, the generated one or more notification may be based on one or more of at least a light signal, a beep, a buzzer, a vibration output, a Short Messaging Service (SMS), a Multimedia Messaging Service (MMS), a video call, a social media notification.

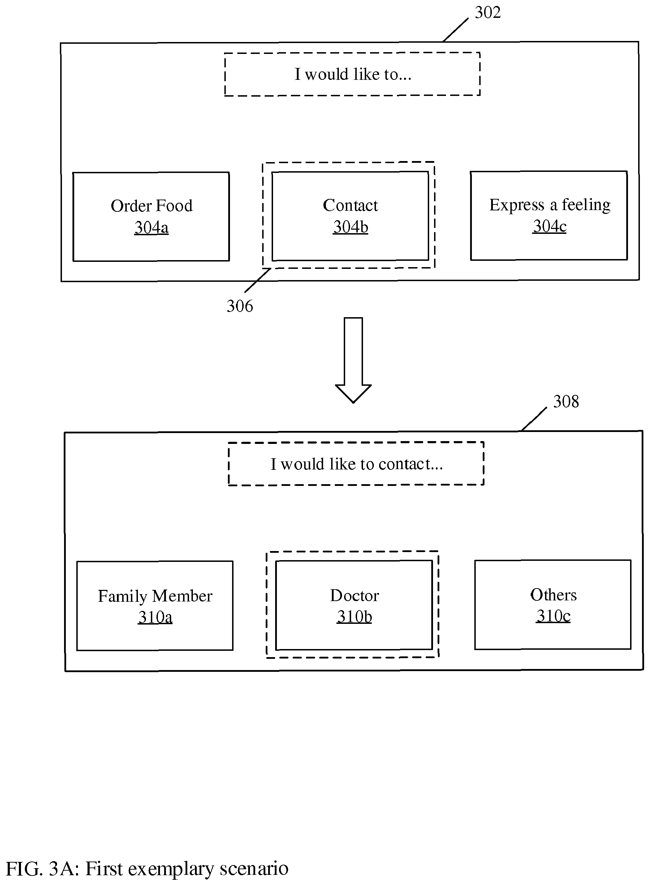

FIG. 3A illustrates a first exemplary scenario of adapting a display on an electronic device, in accordance with some embodiments of the present disclosure. The elements of FIG. 3A have been described in conjunction with elements of FIG. 1 and FIG. 2.

With reference to FIG. 3A, there is shown a first user interface 302 that may be displayed on the display screen 110. The first user interface 302 may include a first plurality of graphical prompts in the form of a first graphical prompt 304a, a second graphical prompt 304b, and a third graphical prompt 304c. The processor 202 may sequentially highlight each of the first plurality of graphical prompts for a first pre-defined time interval that may be stored in the memory 204. Such highlighting has been depicted by the bounding box 306.

In an exemplary scenario, the first user interface 302 may correspond to an interface that provides options to user 102, to order a food item, contact a person, or express a feeling. In an instance, the first graphical prompt 304a may correspond to an option to order a food item, the second graphical prompt 304b may correspond to an option to contact a person, and the third graphical prompt 304c may correspond to an option to express a feeling. Based on a selection of a displayed graphical prompt, the processor 202 may be configured to present a second user interface 308 comprising a second plurality of graphical prompts. Such second plurality of graphical prompts may include graphical prompts present in a pre-defined hierarchy of graphical prompts. The graphical prompts included in the pre-defined hierarchy may correspond to a common category. As an example, when the user 102 selects the second graphical prompt 304b for contacting a person, the processor may refer to list of contacts of the user 102, such as a friend, a family member, a doctor, a lawyer, or a colleague, present in the pre-defined hierarchy. Each of the contact in the hierarchy may be mapped with a graphical prompt that may constitute the second plurality of graphical prompts.

Based on the detection of one or more pre-defined eye-blinking gestures by the image tracking device 112, the processor 202 may instruct the adaptation engine to adapt the first user interface to a second user interface 308. The adaptation may be further performed based on historical selections performed by the user 102.

In an instance, the adapted second user interface 308 may include the second plurality of graphical prompts corresponding to a modified hierarchy. For example, the modified hierarchy of the second plurality of graphical prompts may include graphical prompts 310a for contacting the family member, graphical prompts 310b for contacting the doctor, and graphical prompt 310c for contacting other members in the contact list of the user 102. Such other members may correspond to the friend, the lawyer, and the colleague, that are contacted less frequently. Here, the hierarchy of the pre-defined second plurality of graphical prompts has been modified by the adaptation engine 208, so as to display only those graphical prompts that are frequently selected by the user 102. At the same time, based on the graphical prompt 310c, the user 102 may refer to less frequently used contacts or expand the pre-defined hierarchy by adding a new contact. Such a modification of the pre-defined hierarchy pre-empts the selection input that may be provided by the user 102 and thereby reduces the number of interactions required by the user 102 to select a graphical prompt.

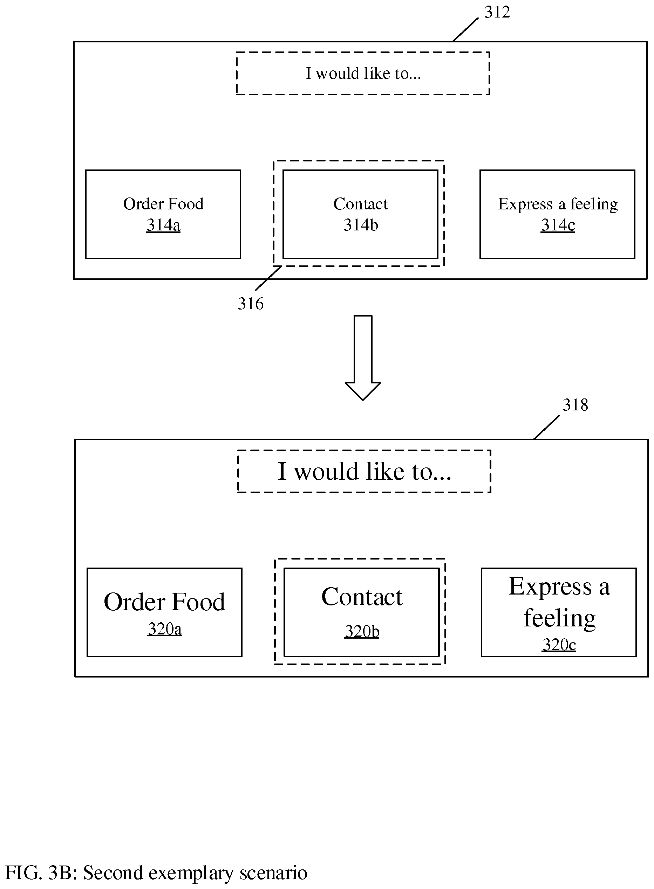

With reference to FIG. 3B, there is shown a first user interface 312 that may be displayed on the display screen 110. The first user interface 312 may include a first plurality of graphical prompts in the form of a first graphical prompt 314a, a second graphical prompt 314b, and a third graphical prompt 314c. The processor 202 may sequentially highlight each of the first plurality of graphical prompts for a first pre-defined time interval that may be stored in the memory 204. Such highlighting has been depicted by the bounding box 316.

In an exemplary scenario, when the one or more pre-defined eye-blinking gestures of the user 102 are not detected for a pre-defined time interval, the adaptation engine 208 may be configured to increase the font size of the displayed graphical prompts. Such graphical prompts having increased font size may be displayed in the second user interface 318 in the form of a first graphical prompt 320a, a second graphical prompt 320b, and a third graphical prompt 320c. As has been discussed in the foregoing disclosure, the font size of the graphical prompt may be changed when no user input is detected for one or more pre-defined cycles of highlighting the first plurality of graphical prompts.

A person of ordinary skill in the art will appreciate that the aforementioned adaptation may further include controlling other display properties of the displayed first plurality of graphical prompts, based on the user inputs. Such display properties have been discussed in detail in the FIGs. 1 and 2.

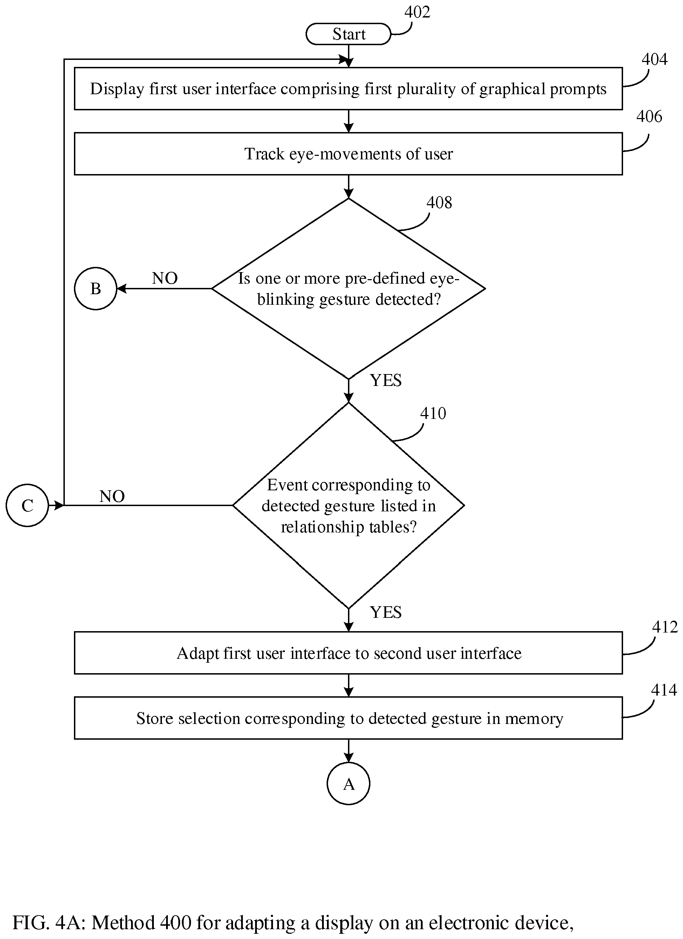

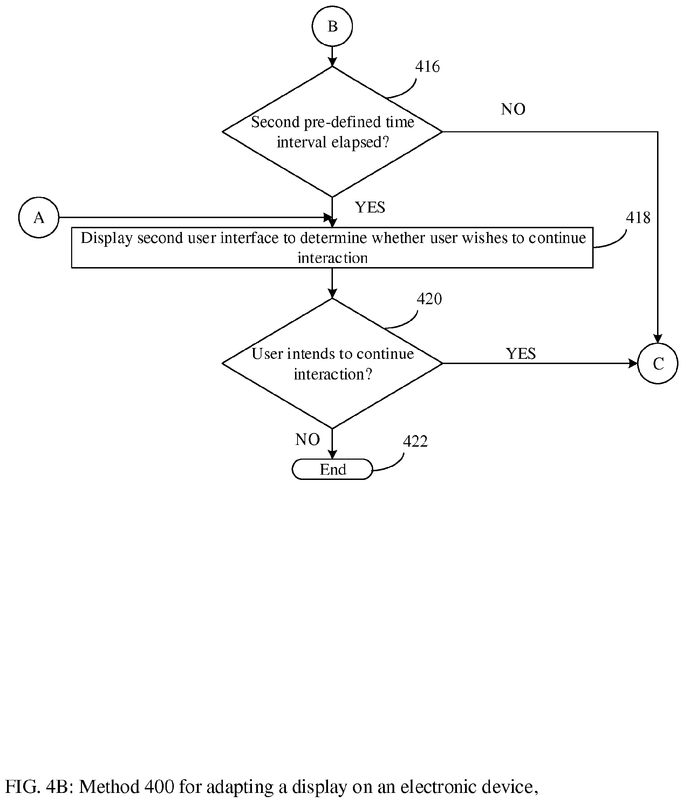

FIG. 4 is a flow diagram of a detailed exemplary process for adapting a display on an electronic device, in accordance with some embodiments of the present disclosure. With reference to FIG. 4, there is shown a flow chart 400. The flow chart 400 is described in conjunction with FIGs. 1 to 3. The process starts at step 402 and proceeds to step 404.

At step 404, a first user interface comprising a first plurality of graphical prompts may be displayed on the display screen 110, by the processor 202. Each of the displayed first plurality of graphical prompts may be highlighted sequentially based on a first pre-defined time interval. At step 406, tracking of the eye-movements of a user (such as the user 102) may be performed based on the image tracking device 112. At step 408, based on the tracking of the eye movements of the user 102, it may be determined one or more pre-defined eye-blinking gesture to select a highlighted graphical prompt of the first user interface are detected. It may be further determined whether such gestures are detected within a second pre-defined time interval. The aforementioned first pre-defined time interval and the second pre-defined time interval may be stored in the memory 204.

In instances, when one or more one or more pre-defined eye-blinking gesture to select a highlighted graphical prompt of the first user interface are detected, the control passes to step 410. In instances, when one or more one or more pre-defined eye-blinking gesture to select a highlighted graphical prompt of the first user interface are not detected, the control passes to step 416.

At step 410, it may be determined whether the detected one or more predefined eye-blinking gestures correspond to an event type listed in the relationship tables stored in the memory 204. In instances, when the determination at step 410 is positive, the first user interface may be adapted to a second user interface based on the adaptation table 204c, at step 412.The second user interface may include a second plurality of graphical prompts, each of which may be highlighted sequentially based on the first pre-defined time interval. Further, such an adaptation may be based on determination of a type of event associated with the user 102 that corresponds to the detected one or more pre-defined eye-blinking gestures. In an embodiment, the adaptation of the first user interface includes controlling display properties of the first plurality of the graphical prompts and the second plurality of the graphical prompts. The details of controlling the display properties have been discussed in detail in FIGs. 1 to 3. At step 414, the detected one or more pre-defined eye-blinking gestures to select a graphical prompt may be stored in the memory 204. The control passes to the step 418. In instances, when the determination at step 410 is negative, the control passes to step 404.

At step 416, it may be determined whether the second pre-defined time interval has elapsed. In instance, when the second pre-defined time interval has elapsed without detection of one or more pre-defined eye-blinking gesture, a second user interface may be displayed to the user 102, by the adaptation engine 208, at step 418. Such a second user interface may be displayed to the user 102 to determine whether the user intends to continue the interaction. Such a determination may be performed at the step 420. In instances, when the user confirms continuation of the interaction, the control passes to step 404. In instances, when the user 102 intends to discontinue the interaction, the control passes to end step 422 where the system may turn off. In an embodiment, when the user 102 intends to discontinue the interaction, the control may pass to the step 404. In such an embodiment, the first user interface may be displayed to the user 102 and a polling may be performed to track eye movements of the user 102. In another embodiment, when the user 102 intends to discontinue the interaction, one or more attendants (such as family, doctor, or a nurse) may be notified. In an instance, based on the notification, the attendants may command the electronic device 104 to switch off. In another instance, the attendants may change the state of the system to an idle state. In the idle state, the first user interface may be displayed to the user 102 and a polling may be performed to track eye movements of the user 102.

As will be also appreciated, the above described techniques may take the form of computer or controller implemented processes and apparatuses for practicing those processes. The disclosure can also be embodied in the form of computer program code containing instructions embodied in tangible media, such as floppy diskettes, CD-ROMs, hard drives, or any other computer-readable storage medium, wherein, when the computer program code is loaded into and executed by a computer or controller, the computer becomes an apparatus for practicing the invention. The disclosure may also be embodied in the form of computer program code or signal, for example, whether stored in a storage medium, loaded into and/or executed by a computer or controller, or transmitted over some transmission medium, such as over electrical wiring or cabling, through fiber optics, or via electromagnetic radiation, wherein, when the computer program code is loaded into and executed by a computer, the computer becomes an apparatus for practicing the invention. When implemented on a general-purpose microprocessor, the computer program code segments configure the microprocessor to create specific logic circuits.

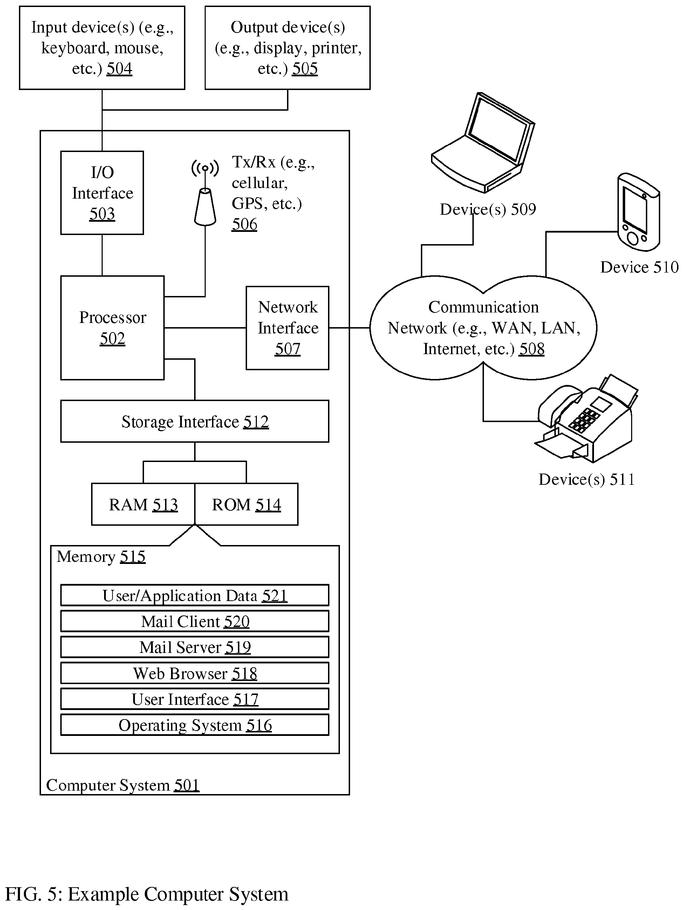

The disclosed methods and systems may be implemented on a conventional or a general-purpose computer system, such as a personal computer (PC) or server computer. Referring now to FIG. 5, a block diagram of an exemplary computer system 501 for implementing embodiments consistent with the present disclosure is illustrated. Variations of computer system 501 may be used for implementing network environment 100 for transmitting data over a communication network. Computer system 501 may include a central processing unit ("CPU" or "processor") 502. Processor 502 may include at least one data processor for executing program components for executing user- or system-generated requests. A user may include a person, a person using a device such as such as those included in this disclosure, or such a device itself. The processor may include specialized processing units such as integrated system (bus) controllers, memory management control units, floating point units, graphics processing units, digital signal processing units, etc. The processor may include a microprocessor, such as AMD Athlon, Duron or Opteron, ARM's application, embedded or secure processors, IBM PowerPC, Intel's Core, Itanium, Xeon, Celeron or other line of processors, etc. The processor 502 may be implemented using mainframe, distributed processor, multi-core, parallel, grid, or other architectures. Some embodiments may utilize embedded technologies like application-specific integrated circuits (ASICs), digital signal processors (DSPs), Field Programmable Gate Arrays (FPGAs), etc.

Processor 502 may be disposed in communication with one or more input/output (I/O) devices via I/O interface 503. The I/O interface 503 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 503, the computer system 501 may communicate with one or more I/O devices. For example, the input device 504 may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc. Output device 505 may be a printer, fax machine, video display (e.g., cathode ray tube (CRT), liquid crystal display (LCD), light-emitting diode (LED), plasma, or the like), audio speaker, etc. In some embodiments, a transceiver 506 may be disposed in connection with the processor 502. The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor 502 may be disposed in communication with a communication network 508 via a network interface 507. The network interface 507 may communicate with the communication network 508. The network interface may employ connection protocols including, without limitation, direct connect, Ethernet (e.g., twisted pair 10/100/1000 Base T), transmission control protocol/internet protocol (TCP/IP), token ring, IEEE 802.11 a/b/g/n/x, etc. The communication network 508 may include, without limitation, a direct interconnection, local area network (LAN), wide area network (WAN), wireless network (e.g., using Wireless Application Protocol), the Internet, etc. Using the network interface 507 and the communication network 508, the computer system 501 may communicate with devices 509, 510, and 511. These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system 501 may itself embody one or more of these devices.

In some embodiments, the processor 502 may be disposed in communication with one or more memory devices (e.g., RAM 513, ROM 514, etc.) via a storage interface 512. The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc.

The memory devices may store a collection of program or database components, including, without limitation, an operating system 516, user interface application 517, web browser 518, mail server 519, mail client 520, user/application data 521 (e.g., any data variables or data records discussed in this disclosure), etc. The operating system 516 may facilitate resource management and operation of the computer system 501. Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. User interface 517 may facilitate display, execution, interaction, manipulation, or operation of program components through textual or graphical facilities. For example, user interfaces may provide computer interaction interface elements on a display system operatively connected to the computer system 501, such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, the computer system 501 may implement a web browser 518 stored program component. The web browser may be a hypertext viewing application, such as Microsoft Internet Explorer, Google Chrome, Mozilla Firefox, Apple Safari, etc. Secure web browsing may be provided using HTTPS (secure hypertext transport protocol), secure sockets layer (SSL), Transport Layer Security (TLS), etc. Web browsers may utilize facilities such as AJAX, DHTML, Adobe Flash, JavaScript, Java, application programming interfaces (APIs), etc. In some embodiments, the computer system 501 may implement a mail server 519 stored program component. The mail server may be an Internet mail server such as Microsoft Exchange, or the like. The mail server may utilize facilities such as ASP, ActiveX, ANSI C++/C#, Microsoft .NET, CGI scripts, Java, JavaScript, PERL, PHP, Python, WebObjects, etc. The mail server may utilize communication protocols such as internet message access protocol (IMAP), messaging application programming interface (MAPI), Microsoft Exchange, post office protocol (POP), simple mail transfer protocol (SMTP), or the like. In some embodiments, the computer system 501 may implement a mail client 520 stored program component. The mail client may be a mail viewing application, such as Apple Mail, Microsoft Entourage, Microsoft Outlook, Mozilla Thunderbird, etc.

In some embodiments, computer system 501 may store user/application data 521, such as the data, variables, records, etc. (e.g., past ticket repository, keywords, Ngrams, clusters or categories, relationship mapping, user queries, resolutions, and so forth) as described in this disclosure. Such databases may be implemented as fault-tolerant, relational, scalable, secure databases such as Oracle or Sybase. Alternatively, such databases may be implemented using standardized data structures, such as an array, hash, linked list, struct, structured text file (e.g., XML), table, or as object-oriented databases (e.g., using ObjectStore, Poet, Zope, etc.). Such databases may be consolidated or distributed, sometimes among the various computer systems discussed above in this disclosure. It is to be understood that the structure and operation of the any computer or database component may be combined, consolidated, or distributed in any working combination.

As will be appreciated by those skilled in the art, the techniques described in the various embodiments discussed above provide for adapting a display on an electronic device, thereby pre-empting the selection inputs that may be provided by the user. Such an operation enables a reduction in the amount of interaction required by the system, with the user. This is particularly desirable when the user is an incapacitated patient and faces difficulty in providing inputs by means of audio or touch-based inputs.

Additionally, the techniques described in the various embodiments discussed above are based on pre-defined eye-blinking gestures of the user. Such pre-defined eye-blinking gestures enhance the usability as such gestures are easier to remember, along with being easily detectable. Also, because of the dependency on the eye-blink based gestures, the system disclosed above is more robust as against a gaze based system. Furthermore, the inherent capability of the system discussed above to generate one or more notifications enables a remote user to stay aware about the real-time status of the user. Such status may correspond to a medical state of the user, and/or the requirements of the user. Further, such updates enable the remote user to timely address any medical emergency associated with the user.

The specification has described system and method for adapting a display on an electronic device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method of adapting a display on an electronic device, the method comprising:
displaying, on a display unit, a first user interface comprising a first plurality of graphical prompts to a user, wherein the first plurality of graphical prompts are highlighted sequentially;
detecting, via an image recognition unit, one or more pre-defined eye-blinking gestures of the user to select a highlighted graphical prompt of the first user interface; and
adapting, by an adaptation engine, the first user interface, to display a second user interface in response to detecting the one or more predefined eye-blinking gestures of the user.

2. The method of claim 1, wherein at least one of the second user interface comprises a second plurality of graphical prompts.

3. The method of claim 2, wherein each of the first plurality of graphical prompts and the second plurality of graphical prompts is highlighted for a first pre-defined time interval.

4. The method of any of the preceding claims, further comprising automatically transitioning from the first user interface to the second user interface based on a second pre-defined time interval, wherein the transitioning comprises at least a vertical scrolling or a horizontal scrolling.

5. The method of any of the preceding claims, wherein the determination of the second user interface is based on one or more historical selections associated with the user.

6. The method of any of the preceding claims, further comprising generating one or more notifications based on the detected one or more pre-defined eye-blinking gestures of the user.

7. The method of claim 6, wherein the generated one or more notifications correspond to one or more of: a medical event associated with the user, a communication corresponding to a physical or a mental state of the user, a status of request placed by the user, a selection of the one or more graphical prompts from the first plurality of graphical prompts.

8. The method of claim 7, wherein the medical event associated with the user is determined by the electronic device, based on at least a comparison of average time taken by the user to select a highlighted graphical prompt, with a pre-defined time threshold.

9. The method of any of the preceding claims, wherein the one or more predefined eye-blinking gesture comprise one or more of at least: a blink of one or both eyes of the user for a pre-defined count, closing one or both eyes of the user for a pre-defined duration, a combination of blink of the eyes of the user.

10. The method of any of the preceding claims, wherein adapting the first user interface comprises controlling display properties of the first plurality of the graphical prompts and the second plurality of the graphical prompts.

11. The method of claim 10, wherein the display properties comprise one or more of at least: a time interval of the sequential highlighting of the first plurality of the graphical prompts, a time interval of a sequential highlighting of the second plurality of the graphical prompts, a size of the first plurality of the graphical prompts or the second plurality of the graphical prompts, a display duration of the first plurality of the graphical prompts or the second plurality of the graphical prompts, a level of brightness of the display unit.

12. The method of any of the preceding claims, further comprising storing the detected one or more one or more pre-defined eye-blinking gestures of the user to select a highlighted graphical prompt, in a memory associated with the electronic device.

13. A system for of adapting a display on an electronic device, the system comprising:
a processor; and
a memory communicatively coupled to the processor, wherein the memory stores the processor-executable instructions, which, on execution, causes the processor to perform the method of any of the preceding claims.

14. A non-transitory computer-readable medium storing instructions for adapting a display based on eye-based user inputs, wherein upon execution of the instructions by one or more processors, the processors perform the method of any of claims 1 to 12.
